# EUROPEAN PATENT APPLICATION

(11) **EP 3 130 327 A1**
(43) Date of publication of application: **15.02.2017**
(21) Application number: 16192093.9
(22) Date of filing: 19.12.2011
(51) Int. Cl.: A61K 8/38, A61K 8/64, A61Q 11/00

(54) **SYSTEM PROVIDING ENZYME-CATALYZED REACTION**

(62) Divisional of application: 11804898.2
(71) Applicant: Colgate-Palmolive Company, New York, NY 10022 (US)
(72) Inventor: BOYD, Thomas, Metuchen, NJ New Jersey 08840 (US); XU, Guofeng, Plainsboro, NJ New Jersey 08536 (US); ADAMS, Richard, Monmouth Junction, NJ New Jersey 08852 (US); PIERCE, Robert, Basking Ridge, NJ New Jersey 07920 (US); SAMAROO, Derek, Edison, NJ New Jersey 08820 (US); VISCIO, David, Prescott Valley,, AZ Arizona 86315 (US)
(74) Representative: Jenkins, Peter David

(57) **Abstract**

Described herein are packages for storing and dispensing multi-part tooth whitening formulations, wherein the contents of the parts are mixed, a peracid and/or dioxirane whitening agent is formed. Particular multi-part tooth whitening formulations using this principle and methods of use thereof are also provided.

## Description

### BACKGROUND

It is frequently desirable to keep formulation components separate prior to use, for example because the components may be too unstable for long-term storage if combined. It is desirable in such cases to be able to mix the formulation components at the point of use in an efficient and simple way.

One example of a formulation where it may be desirable to keep formulation components separate is tooth-whitening formulations comprising reactive ingredients such as peroxides or peroxyacids or their precursors. For example, one may want to combine A+B or A+B+C to obtain an unstable whitening composition X, but keep A and B separate up to that point. The difficulty arises in that during use the mixing must be rapid, and diffusion of the whitening composition, X, to the tooth surface must be efficient. Unfortunately, combining multiple gels or other moderately viscous materials is not generally an efficient way to quickly mix chemicals; if a typical consumer were to mix by hand, it would lead to regions of well-mixed and poorly-mixed sample. One has only to hand-mix two viscous house paints together to easily see the problem: rather than efficient blending of the two colors, laminar flow causes the colors to exist in adjacent streaks. To overcome this problem directly would require more time and mixing effort than the typical user would be willing to devote to the task, and where the reactive species X begins to break down within minutes, such a method would be unworkable.

There is thus a need for products that permit ingredients to be efficiently and effectively combined at the point of use.

### SUMMARY

Some embodiments of the present invention provide a multi-chamber system, wherein one chamber contains a low viscosity liquid solution and another contains a liquid, powder or mixture of powders, the chambers being separated by a frangible or tearable barrier, such that upon squeezing one chamber, the barrier breaks and the components of the chambers can mix, to form a solution, emulsion, suspension or extrudable gel, which can be dispensed through an outlet in the second chamber, wherein the contents of the chambers, upon mixing, provide a peracid and/or a dioxirane.

For example, one chamber may contain a low viscosity liquid solution comprising a protein having perhydrolase activity, while the other chamber or chambers contains a carboxy donor, e.g., a carboxylic acid or acyl compound, and a peroxide source, such that upon mixing of the contents of the chambers, the protein having perhydrolase activity catalyzes a reaction between the peroxide released by the peroxide source and the carboxy donor to form a peracid. Applied to the teeth, such a peracid is highly effective for whitening teeth, so that effective whitening action can be achieved in a shorter period and with lower peroxide levels.

In a particular embodiment, one chamber contains a low viscosity aqueous solution comprising a protein having perhydrolase activity, and another chamber contains a gellant, a peroxide, and an acetyl-containing compound, all in powder form, such that when the barrier is broken and the contents of the chambers allowed to mix, the peroxide and the acetyl containing compound can react, the reaction being catalyzed by the perhydrolase, to form peracetic acid, in an extrudable gel formed by the liquid and the gellant, which extrudable gel can then be extruded and applied to the teeth, e.g., using a tray or strip, for sufficient time, e.g., 10-30 minutes, to whiten one or more teeth.

In some embodiments, the peracid provided by the enzyme-catalyzed reaction of peroxide and carboxy donor as described reacts with a ketone to provide a dioxirane, which forms the whitening agent in the extrudable gel.

In other embodiments, one chamber comprises a peracid and another chamber comprises a ketone, such that upon mixing, the peracid reacts with the ketone to provide the corresponding dioxirane, which forms the whitening agent in the extrudable gel.

Further areas of applicability of the present invention will become apparent from the detailed description provided hereinafter. It should be understood that the detailed description and specific examples, while indicating the preferred embodiments of the invention, are intended for purposes of illustration only and are not intended to limit the scope of the invention.

### BRIEF DESCRIPTION OF THE DRAWINGS

The present invention will become more fully understood from the detailed description and the accompanying drawings, wherein:

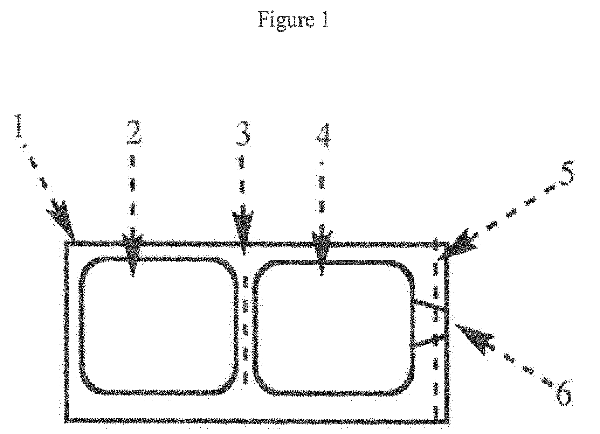

Figure 1 depicts an embodiment of the invention which is a two-chambered package in accordance with the invention, the package being heat-sealed about the perimeter (1), and having a first chamber (2) which contains a liquid component and a second chamber (4) comprising a powder component, separated by a frangible seal (3), such that when the first chamber (2) is squeezed, the frangible seal (3) ruptures and the liquid flows into the second chamber (4) and mixes with the powder, which resulting mixture can then be dispensed by breaking the scored edge (5) to allow the mixture to flow or be squeezed out of the nozzle (6).

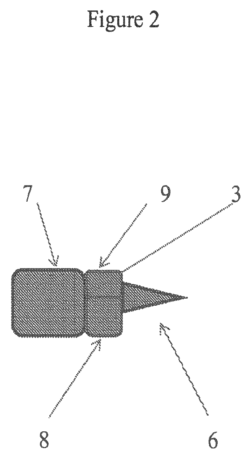

Figure 2 depicts another embodiment of the invention, permitting mixing of components just prior to use, as described for Figure 1, but utilizing a three-chambered package having a nozzle which can be opened by the consumer for dispensing product. In this embodiment, the package comprises a first chamber (7), a second chamber (8), a third chamber (9), the chambers being separated by frangible seals (3), and a nozzle with a break-away tip (6) to dispense the materials after mixing.

### DETAILED DESCRIPTION

The following description of the preferred embodiment(s) is merely exemplary in nature and is in no way intended to limit the invention, its application, or uses.

In some embodiments, the invention provides a package for an oral care product which comprises multiple chambers and is designed to keep the ingredients in each chamber separate and non-reactive until the point of use. For example, some embodiments provide a chemically stable structural package design which permits an enzyme catalyzed tooth whitening product to reach pre-steady state kinetics in milliseconds after the ingredients are exposed to each other and mixed. The contents of the container are dispensed via an opening means, e.g., through a nozzle with a removable cap or plug or which becomes functional when a preferentially scored section of the container is broken off by the consumer, permitting clean and convenient dispensing of product through a shaped nozzle.

In some embodiments, the chambers have the capacity to store, e.g., 0.1 - 30 grams of an ingredient. In some embodiments, the oral care product is a tooth whitening product providing a total quantity of product delivered from all chambers, e.g., between 1.0 to 5.0 grams, for example 1-2 grams to provide the intended benefit. The volumetric capacity of the chambers is designed to accommodate ingredients with a specific gravity of e.g.,1.0 to 1.1 and preferably with a specific gravity range of 1.02 to 1.05.

In some embodiments, the package is manufactured using a thermoforming process of at least two flexible films with a thickness of 50 micron to 500 micron and preferably 300 micron thick. The two films may be opaque, translucent or transparent and can be any combination when assembled in the thermoforming process. Both materials provide water vapor barrier characteristics, e.g., with less than 3% moisture loss over a three year time frame, e.g., less than 1% moisture loss over the same period. The films also provide a flavor barrier. The flavor loss can be determined both by gas chromatography and by organoleptic evaluation.

The films are chemically resistant to the materials comprised therein. For example, in some embodiments, they are resistant to 0.1 % to 10% hydrogen peroxide solution by weight, e.g. up to 0.3% hydrogen peroxide solution by weight.

In some embodiments, one of the two flexible materials is a polymeric laminate and the inner layer of the laminate has been selected to bond with the first flexible material but will delaminate when pressure is manually applied to the chamber with a frangible seal. The force required to break the seal is manually applied and can vary between 2 inch-lbf and 5 inch-lbf.

After the frangible seal between the compartments are broken (or compromised), the ingredients in each chamber will come into intimate contact with each other. The consumer is permitted to mix the individual ingredients for a period of time to exceed the pre-steady state kinetic rate or the burst phase. The time for pre-steady state kinetics or burst phase can be in milliseconds. This provides sufficient time for the formation and consumption of enzyme-substrate intermediates until their steady state concentrations are reached. After steady state has been achieved, the consumer can break a preferentially scored section of the multi chamber container and dispense the mixture onto a dental tray. The tray is applied to the teeth for a period of time of 15 minutes to 45 minutes and provides an effective whitening benefit.

Exemplary embodiments of the invention thus include for example packages, oral care compositions, and methods of whitening teeth, e.g.:
1. Package 1, a package comprising a deformable material configured to form at least two sealed chambers, the package having
   (i) a first chamber, a second chamber, and optionally additional chambers being separated by one or more barriers which are frangible or tearable, at least one of which chambers contains a low viscosity liquid, wherein one or more barriers is compromised or breaks (e.g., as a result of squeezing the chamber with the low viscosity liquid), the contents of the chambers mix and react to provide a mixture comprising a peracid and/or a dioxirane whitening material, and
   (ii) an opening means, for example a scored region, cap or plug to allow opening of the package, to provide an outlet through which the mixture can be dispensed.

   1.1. Package 1 wherein the first chamber contains a low viscosity liquid solution comprising a protein having perhydrolase activity, a second chamber contains a carboxy donor, e.g., a carboxylic acid or acyl compound, and the second or and optional additional chamber contains a peroxide source, such that upon squeezing the first chamber, one or more barriers between the chambers breaks permitting the low viscosity liquid solution to mix with the peroxide source and the carboxy donor, whereupon the low viscosity liquid solution comprising a protein having perhydrolase activity catalyzes a reaction between the peroxide released by the peroxide source and the carboxy donor to form a peracid.
   1.2. Any of the foregoing packages wherein one of the chambers contains a low viscosity aqueous solution and another contains a gellant, such that upon mixing and formation of the peracid and/or dioxirane, an extrudable gel is formed by the liquid and the gellant, which extrudable gel can then be extruded and applied to the teeth, e.g., using a tray or strip, for sufficient time, e.g., 10-30 minutes, to whiten one or more teeth.
   1.3. Any of the foregoing packages wherein one chamber comprises as the low viscosity aqueous liquid wherein the liquid is a solution of a protein having perhydrolase activity, and another chamber contains a gellant, a peroxide, and an acetyl-containing compound, all in powder form, such that when the barrier is broken and the contents of the chambers allowed to mix, the peroxide and the acetyl containing compound can react, the reaction being catalyzed by the perhydrolase, to form peracetic acid, in an extrudable gel.
   1.4. Any of the foregoing packages wherein one or more of the chambers contains a ketone which will react to form a dioxirane in the presence of a peracid.
   1.5. The foregoing package wherein a peracid provided by the enzyme-catalyzed reaction of peroxide and carboxy donor reacts with the ketone to provide a dioxirane.
   1.6. Package 1.4 or 1.5 wherein one chamber comprises a peracid and another chamber comprises a ketone, such that upon mixing, the peracid reacts with the ketone to provide the corresponding dioxirane.
   1.7. Any of the foregoing packages containing a ketone wherein the ketone is methyl ethyl ketone.
   1.8. Any of the foregoing packages wherein the deformable material is plastic or aluminum.
   1.9. Any of the foregoing packages wherein the low viscosity liquid solution has a viscosity sufficiently low to ensure efficient mixing with the contents of the second chamber, e.g., below 5,000 cps, e.g. below 500 cps.
   1.10. Any of the foregoing packages wherein the low viscosity liquid solution comprises a buffer.
   1.11. Any of the foregoing packages containing a carboxy donor which is reactive with a peroxide in the presence of a perhydrolase to provide a peracid, and which is selected from (i) one or more C₂₋₁₈ carboxylic acids, e.g C₂₋₆ carboxylic acids (e.g., acetic acid), including lower linear or branched alkyl carboxylic acids, optionally substituted with hydroxy and/or C₁₋₄ alkoxy; (ii) one or more hydrolysable and acceptable esters thereof (e.g. mono-, di-, and tri-glycerides and acylated saccarides) and (iii) mixtures thereof.
   1.12. Any of the foregoing packages containing a carboxy donor which is reactive with a peroxide in the presence of a perhydrolase to provide a peracid, and which is selected from 1,2,3-triacetoxypropane (sometimes referred to herein as triacetin or glycerin triacetate) and acylated saccharides, e.g. acetylated saccharides.
   1.13. Any of the foregoing packages comprising a carboxy donor which is reactive with a peroxide in the presence of a perhydrolase to provide a peracid, and which comprises an ester compound having solubility in water of at least 5 ppm at 25 °C.
   1.14. Any of the foregoing packages containing a peroxide source wherein the peroxide source is selected from solid peroxides and solid peroxide donors and mixtures thereof, e.g., selected from peroxide salts or complexes (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate); hypochlorites; urea peroxide; hydrogen peroxide polymer complexes such as hydrogen peroxide-polyvinyl pyrrolidone polymer complexes; metal peroxides e.g. zinc peroxide and calcium peroxide; for example a solid peroxide selected from urea peroxide, polyvinylpyrrolidone-hydrogen peroxide complexes, sodium percarbonate, sodium perborate, and metal peroxides e.g. zinc peroxide and calcium peroxide.
   1.15. The foregoing package wherein the peroxide source is urea peroxide.
   1.16. Any of the foregoing packages wherein at least one of the chambers contains an orally acceptable ketone, e.g., a C₃₋₈ alkyl ketone compound, for example methyl ethyl ketone, wherein upon upon mixing of the contents of the first and second chambers and formation of the peracid, the ketone reacts with the peracid to form the corresponding dioxirane.
   1.17. Any of the foregoing packages wherein the ingredients of the chambers are present in amounts sufficient to provide, upon mixing, a whitening agent in an amount and concentration effective to whiten teeth.
   1.18. Any of the foregoing packages wherein the first chamber contains the low viscosity liquid and the second chamber contains a gellant in powder form.
   1.19.The foregoing package wherein the gellant is selected from carbomer gellants (e.g., Carbopol 971P), polysaccharide gums, such as xanthan gum, modified food starches, animal or fish-based gelatin, and silicas.
   1.20. The foregoing package wherein the gellant is a carbomer gellant.
   1.21. Any of the foregoing packages wherein the second chamber contains a gellant in powder form in a relative amount to provide a viscosity of 100,000 to 150,000 cps, e.g., about 125,000 cps, upon mixing with the contents of the first chamber, e.g., wherein the gellant is present in amounts of from 5% to 50% by weight of the final mixture of the contents of the first and second chambers.
   1.22. Any of the foregoing packages wherein the first chamber contains a low viscosity aqueous solution comprising a protein having perhydrolase activity and a buffer, and the second chamber contains a gellant, a peroxide, and an acetyl-containing compound, all in powder form, such that when the frangible barrier is broken and the contents of the two chambers allowed to mix, the peroxide and the acetyl containing compound can react, the reaction being catalyzed by the protein having perhydrolase activity, to form peracetic acid, in an extrudable gel formed by the liquid and the gellant, which extrudable gel can then be extruded and applied to the teeth, e.g., using a tray or strip, for sufficient time, e.g., 10-30 minutes, to whiten one or more teeth.
   1.23.Any of the foregoing packages which further comprises an applicator device such as a dental tray or strip for applying a mixture of the contents of the first and second chambers to the teeth.
   1.24. The foregoing package wherein, when the mixture is dispensed, the opening from the second chamber is directly attached to a tray so that the mixture is extruded into the tray.
2. Composition 2, being a multi-part oral care composition comprising a first part which is physically separated from a second part during storage and combined with the second part just prior to use, e.g., within 10 minutes of use, wherein the parts comprise ingredients that, when combined, provide a peracid and/or dioxirane whitening material, e.g.,
   2.1. Composition 2 wherein the first part comprises protein having perhydrolase activity as described for any of the foregoing packages, and second part comprises a peroxide source and a carboxy donor selected from carboxylic acids and acyl compounds, wherein the peroxide source and the carboxy donor react in the presence of the perhydrolase to form a peracid, e.g., a peroxide source and a carboxy donor as described for any of the foregoing packages, e.g.,
   2.2. The foregoing composition wherein the carboxy donor is selected from C2-18 carboxylic acids (e.g., acetic acid), and hydrolysable and acceptable esters thereof (e.g. mono-, di-, and tri-glycerides) and mixtures thereof 2.3. The foregoing composition wherein the carboxy donor is 1,2,3-triacetoxypropane (sometimes referred to herein as triacetin or glycerin triacetate).
   2.4. Any of the foregoing compositions wherein the peroxide source is a solid peroxide selected from urea peroxide, polyvinylpyrrolidone-hydrogen peroxide complexes, sodium percarbonate, sodium perborate, and metal peroxides e.g. zinc peroxide and calcium peroxide.
   2.5. Any of the foregoing compositions wherein the peroxide source is urea peroxide.
   2.6. Any of the foregoing compositions wherein the first or second part comprises an orally acceptable ketone, e.g., methyl ethyl ketone, e.g., which forms a dioxirane upon reaction with a peracid, e.g., wherein the first part comprises an orally acceptable ketone which forms a dioxirane upon reaction with a peracid, and the second part comprises a peracid (e.g., an imido-alkane-percarboxylic acid, for example 6-phthalimidoperoxyhexanoic acid) or a peracid source (e.g., a peroxide and a carboxy dononr which react in the presence of a perhydolase to form a peracid).
   2.7. Any of the foregoing compositions when packaged in a package as hereinbefore described, e.g. Package 1 et seq.
3. A method (Method 3) of whitening teeth comprising activating a multi-part oral care composition as hereinbefore described, by combining the two parts, and applying an effective amount of the mixture thus obtained to the teeth, e.g., using an applicator, for example a dental tray or a strip, for a sufficient time, e.g., at least 10 minutes, for example 10-30 minutes, to whiten the teeth.

Peroxycarboxylic acids ("peracids") are known as effective antimicrobial and whitening agents. U.S. Patent 5,302,375 to Viscio, D., discloses oral compositions for whitening comprising peracetic acid dissolved in a vehicle, wherein the peracetic acid is generated within the vehicle in situ by combining water, acetylsalicylic acid, and a water soluble alkali metal percarbonate. U.S. Patent 5,279,816 to Church et al. discloses the use of a composition comprising peracetic acid to whiten stained or discolored teeth. U.S. Patents 6,221,341 and 7,189,385 to Montgomery, R., disclose peroxy acid tooth-whitening compositions suitable for use in a method to whiten teeth. More specifically, a peracetic acid composition may be produced by combining a hydrogen peroxide precursor, an acetic acid ester of glycerin, and water to generate, via chemical perhydrolysis, peracetic acid.

Enzymatic perhydrolysis is not described in these references. U.S. Patent Application Publication No. 2009-0311198 to Concar et al. discloses an oral composition comprising a *M*. *smegmatis* enzyme having perhydrolytic activity to bleach teeth.

Many hydrolases and esterases, for example, lipases, serine hydrolases and carbohydrate esterases, catalyze perhydrolysis, the reversible formation of peracids from carboxylic acids and hydrogen peroxide. Perhydrolases, esterases, and lipases generally contain a catalytic triad consisting of a serine (Ser), a glutamate (Glu) or aspartate (Asp), and a histidine (His). Many perhydrolases (e.g. metal-free haloperoxidases) contain a Ser-His-Asp catalytic triad and catalyze the reversible formation of peracid from hydrogen peroxide and carboxylic acids. Without being bound by theory, it is believed that perhydrolysis takes place with an esterase-like mechanism in which a carboxylic acid reacts with the active site serine to form an acyl enzyme intermediate, which then reacts with hydrogen peroxide to form a peracid.

Numerous perhydolases have been described in the art. The inclusion of specific variant subtilisin Carlsberg proteases having perhydrolytic activity in a body care product is disclosed in U.S. Patent 7,510,859 to Wieland et al. Perhydrolytic enzymes beyond the specific variant proteases are not described nor are there any working examples demonstrating the enzymatic production of peracid as a personal care benefit agent. U.S. Patent Application Publication Nos. 2008-0176783 A1; 2008-0176299 A1; 2009-0005590 A1; and 2010-0041752 A1 to DiCosimo et al. disclose enzymes structurally classified as members of the CE-7 family of carbohydrate esterases (i.e., cephalosporin C deacetylases [CAHs] and acetyl xylan esterases [AXEs]) that are characterized by significant perhydrolytic activity for converting carboxylic acid ester substrates (in the presence of a suitable source of peroxygen, such as hydrogen peroxide) into peroxycarboxylic acids at concentrations sufficient for use as a disinfectant and/or a whitening agent. Some members of the CE-7 family of carbohydrate esterases have been demonstrated to have perhydrolytic activity sufficient to produce 4000 - 5000 ppm peracetic acid from acetyl esters of alcohols, diols, and glycerols in 1 minute and up to 9000 ppm between 5 minutes and 30 minutes once the reaction components were mixed (DiCosimo et al., U.S. 2009-0005590 A1). U.S. Patent application publication No. 2010-0087529 A1 describes variant CE-7 enzymes having improved perhydrolytic activity.

Carboxy donors in the present invention are selected from (i) one or more C₂₋₁₈ carboxylic acids, e.g C₂₋₆carboxylic acids (e.g., acetic acid), including lower linear or branched alkyl carboxylic acids, optionally substituted with hydroxy and/or C₁₋₄ alkoxy; (ii) one or more hydrolysable and acceptable esters thereof (e.g. mono-, di-, and tri-glycerides and acylated saccarides) and (iii) mixtures thereof. For example, carboxy donors include 1,2,3-triacetoxypropane (sometimes referred to herein as triacetin or glycerin triacetate) and acylated saccharides, e.g. acetylated saccharides. In a particular embodiment, esters for this use may, for example, be esters having solubility in water of at least 5 ppm at 25 °C.

The carboxy donors or other materials may optionally be encapsulated. There are a variety of encapsulation options well-known to the art, both natural and synthetic. Modified starches and gum arabic are particularly well-suited since they are food grade, relatively inexpensive, quick to dissolve, and can adsorb fairly high levels of liquid oils. Any impact on the final viscosity needs to be considered.

As noted above, the invention may comprise gellants, for example carbomer gellants (e.g., Carbopol 971P), polysaccharide gums, such as xanthan gum, modified food starches, animal or fish-based gelatin, and silicas. Adhesive gel formulations for use with tooth whitening agents are known in the art, e.g. as described in US Patents 7,862,801; 5,746,598; 6,730,316; 7,128,899. The gellant is useful to thicken whitening solutions to a point where they will not run out of a dental tray or away from the teeth to soft tissue areas. This allows the whitening agent to stay in contact with the teeth for extended periods of time and protects soft tissues. The use of a dental tray and a viscous whitening agent allows a low concentration whitening agent to effectively whiten a person's teeth over a 1-2 week period of time with minimal risk to the patient. Gellants for this use should be selected and adjusted to provide a viscosity upon application of 100,000 to 150,000 cps, e.g., about 125,000 cps,

In a particular embodiment, the package or multi-part composition as hereinbefore described comprises a carbomer gellant, for example a modified polyacrylic acid hydrophilic polymer such as CARBOPOL® manufactured by Lubrizol. Carbomers are capable of forming viscous gels at concentrations above as little as 5% by weight.

In some embodiments of the invention, peracids for reaction with ketones to provide dioxiranes are used. The ketones are for example lower alkyl ketones, for example methylethyl ketones. The peracids for reaction with ketones may be peracids generated by the peroxidase calatalyzed reaction of a carboxy donor and a peroxide as described above, or may be included in the original pre-mixed contents of the package chambers, e.g., provided as dry granules comprising a peracid, e.g., an imido-alkane-percarboxylic acid, for example 6-phthalimidoperoxyhexanoic acid (PAP).

All ingredients for use in the formulations described herein should be orally acceptable. As used herein, the term "orally acceptable" refers to an ingredient or composition which is not unsafe, unpalatable, or otherwise unsuitable for use in the oral cavity.

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### EXAMPLES

### Example 1: Whitening formulations with enzyme-activated triacetin

In a two-chambered package, 1.0 mL of pH 7 phosphate buffer containing 0.04 mg perhydrolase enzyme is stored separately from a multi-component powder. The multi-component powder is illustrated in Tables 1A, 1B, and 1C, and comprises the encapsulated triacetin & flavor, granular urea peroxide, and a carbomer gellant. The ratio of well-blended powders, 1A:1B:1C, in this example is 92.3:1.7:6. The two chambers are separated with a water impermeable heat-sealed barrier which is less strong than the outer seals around the package (see Figure 1). To prepare for use, the consumer presses on the buffer/enzyme chamber, which breaks the frangible internal seal and pushes the buffer/enzyme into the powder chamber. The powders rapidly mix with the liquid, dissolving the peroxide source, the starch with adsorbed triacetin & flavor, and, more slowly, hydrating the gellant. After several seconds of mixing these components, the gel has effectively formed, and is ready to be applied to a tray. Approximately 0.5 grams of the newly-formed gel is applied to both an upper and lower delivery device, yielding a dose of 4.3 mg urea peroxide (equivalent to 1.5 mg hydrogen peroxide), 10 mg triacetin, and 0.01 mg hydrolase enzyme.

Opening a hole in the package, via a pre-scored opening (see Figure 1), the user can apply the gel to a tray, and then wear the tray for 20-30 minutes. Alternatively, the gel can be applied to a flexible strip such as a non-porous flexible polyethylene or a slowly dissolvable film.

**TABLE 1A - Encapsulated triacetin**

| **Ingredient** | **Weight %** |
|---|---|
| Starch (CAPSUL®, National Starch) | 94.6 |
| Triacetin | 4.3 |
| Flavor | 1.1 |
| Total | 100 |

**TABLE 1B - Peroxide**

| **Ingredient** | **Weight %** |
|---|---|
| Urea peroxide granules, 5-10 microns | 100 |

**TABLE 1C - Solid Gellant**

| **Ingredient** | **Weight %** |
|---|---|
| Carbomer gellant (CARBOPOL® 971 P, Lubrizol) | 100 |

### Example 2: Whitening formulation with peracid-activated ketone

Using the same type of packaging as described in Example 1 and either a strip or tray delivery form, the following mixture is prepared. The first chamber contains 0.75 ml liquid of Table 2A. The second chamber contains 0.25 g of a mixture of powders of Table 2B/2C/2D in approximately equal parts. During mixing the user combines 0.75 mL of liquid of Table 2A with 0.25 grams of powder of Table 2B/2C/2D. During mixing the ketone is activated by the peracid to form the corresponding highly-reactive dioxirane.

**TABLE 2A - Liquid**

| **Ingredient** | **Weight %** |
|---|---|
| Water | 99.5 |
| Methyl ethyl ketone | 0.5 |
| Total | 100 |

**TABLE 2B - Peracid**

| **Ingredient** | **Weight %** |
|---|---|
| 6-phthalimidoperoxyhexanoic acid (EURECO® granules, Solvay) | 100 |

**TABLE 2C - Encapsulated flavor**

| **Ingredient** | **Weight %** |
|---|---|
| Gum arabic | 85 |
| Flavor oil | 15 |
| Total | 100 |

**TABLE 2D - Solid gellant**

| **Ingredient** | **Weight %** |
|---|---|
| Carbomcr gellant (CARBOPOL® 971P, Lubrizol) | 100 |

Building on this proof of concept, methyl ethyl ketone to obtain a 0.5% solution is added to the 1.0 mL of pH 7 phosphate buffer containing 0.04 mg perhydrolase enzyme of the formulation of Example 1, and upon activation by breaking the seal between the chamber with the liquid and the chamber with the powders and mixing of the contents of the chambers, dioxirane is produced by peracetic acid formed the hydrolase catalyzed reaction of peroxide and triacetin.

In particular, the invention as described herein provides the following embodiments:

### CLAUSES

1. A package comprising a deformable material configured to form at least two sealed chambers, the package having
   (i) a first chamber, a second chamber, and optionally additional chambers, the chambers being separated by one or more barriers which are frangible or tearable, wherein at least one of said chambers contains a low viscosity liquid, and wherein when one or more barriers between the chambers is compromised, the contents of the chambers mix and react to provide a mixture comprising a peracid and/or a dioxirane whitening material; and
   (ii) an opening means to provide an outlet through which the mixture can be dispensed.
2. The package of clause 1 wherein the first chamber contains a low viscosity liquid solution comprising a protein having perhydrolase activity, the second chamber contains a carboxy donor, and the second chamber contains a peroxide source, such that upon the exertion of force on the package, one or more barriers between the chambers is compromised to an extent sufficient to permit the low viscosity liquid solution to mix with the peroxide source and the carboxy donor, whereupon the low viscosity liquid solution comprising a protein having perhydrolase activity catalyzes a reaction between the peroxide released by the peroxide source and the carboxy donor to form a peracid.
3. The package of clause 1 or clause 2, wherein one of the chambers contains a low viscosity aqueous solution and another contains a gellant, such that upon mixing and formation of the peracid and/or dioxirane, an extrudable gel is formed by the liquid and the gellant, comprising the peracid and/or dioxirane, which extrudable gel can then be extruded and applied to a tooth surface, for sufficient time to whiten the tooth.
4. The package of any of the foregoing clauses wherein one chamber comprises the low viscosity aqueous liquid, wherein the liquid is a solution of a protein having perhydrolase activity, and another chamber contains a gellant, a peroxide, and an acetyl-containing compound, all in powder form, such that when the barrier is compromised and the contents of the chambers are allowed to mix, the peroxide and the acetyl containing compound can react, the reaction being catalyzed by the perhydrolase, to form peracetic acid, in an extrudable gel.
5. The package of any of the foregoing clauses wherein one or more of the chambers contains a ketone which will react to form a dioxirane in the presence of a peracid.
6. The package of any of the foregoing clauses wherein the material in the second chamber is in the form of a powder.
7. The package of any of the foregoing clauses wherein the carboxy donor is selected from one or more of (i) C₂₋₁₈ carboxylic acids, optionally substituted with hydroxy and/or C₁₋₄ alkoxy, (ii) hydrolysable and acceptable esters thereof, and (iii) mixtures thereof.
8. The package of clause 6 wherein the carboxy donor comprises 1,2,3-triacetoxypropane.
9. The package of any of the foregoing clauses wherein the peroxide source is a solid peroxide source selected from urea peroxide, a polyvinylpyrrolidone-hydrogen peroxide complex, sodium percarbonate, sodium perborate, and a metal peroxide.
10. The package according to any of the foregoing clauses wherein the first chamber comprises an orally acceptable ketone, wherein the ketone forms the corresponding dioxirane upon reaction with a peracid.
11. The package according to any of the foregoing clauses wherein the second chamber contains a gellant in powder form.
12. The package according to Clause 10 wherein the gellant is selected from: a carbomer gallant; a polysaccharide gum; a modified food starch; an animal or fish-based gelatin; silica; and a combination of two or more thereof.
13. The package according to any of the foregoing clauses wherein
   a. the low viscosity aqueous solution comprises a perhydrolase and a buffer, and
   b. the second chamber contains a gellant, a peroxide, and an acetyl-containing compound, all in powder form,
   such that when the frangible barrier is compromised and the contents of the chambers are allowed to mix, the peroxide and the acetyl containing compound react in the presence of the perhydrolase to form peracetic acid, in an extrudable gel formed by the liquid and the gellant.
14. The package according to any of the foregoing clauses wherein the mixture of the contents of the chambers forms an orally acceptable extrudable gel.
15. The package according to any of the foregoing clauses wherein the mixture of the contents of the chambers is a palatable substance.
16. The package according to any of the foregoing clauses wherein one or more of the chambers contains a flavoring material.
17. The package according to any of the foregoing clauses wherein one or more of the chambers contains a polymer which adheres to the tooth or gum surface.
18. A multi-part oral care composition comprising a first part which is physically separated from a second part during storage and combined with the second part just prior to use, wherein the parts comprise ingredients that, when combined, provide a peracid and/or dioxirane whitening material.
19. The composition of Clause 18 wherein the first part comprises protein having perhydrolase activity as described for any of the foregoing packages, and the second part comprises a peroxide source and a carboxy donor selected from a carboxylic acid and an acyl compound, wherein the peroxide source and the carboxy donor react in the presence of the perhydrolase to form a peracid.
20. The oral care composition according to clause 19 wherein the carboxy donor is 1,2,3-triacetoxypropane.
21. The oral care composition according to clause 19 or clause 20 wherein the peroxide source is a solid peroxide source selected from urea peroxide, a polyvinylpyrrolidone-hydrogen peroxide complex, sodium percarbonate, sodium perborate, a metal peroxide, and a combination of two or more thereof.
22. The oral care composition according to Clause 21 wherein the peroxide source is urea peroxide.
23. The oral care composition according to any of clauses 18-22 wherein the first part comprises an orally acceptable ketone which forms a dioxirane upon reaction with a peracid.
24. The oral care composition according to any of clauses 18-23 wherein the mixture of the ingredients from the parts forms an orally acceptable extrudable gel.
25. The oral care composition according to any of clauses 18-24 wherein the mixture of the ingredients from the parts is a palatable substance.
26. The oral care composition according to any of clauses 18-25 wherein one or more of the parts contains a flavoring material.
27. The oral care composition according to any of clauses 18-26 wherein one or more of the parts contains a polymer which adheres to the tooth or gum surface.
   A method of whitening teeth comprising:
   a. activating a package according to clause 1 or a multi-part oral care composition according to clause 18 by combining the materials in the different chambers or parts respectively; and
   b. applying an effective amount of the mixture thus obtained to a tooth surface for a sufficient time to whiten a tooth.
28. A method of whitening teeth comprising:
   a. activating a package according to claim 1 or a multi-part oral care composition according to claim 18 by combining the materials in the different chambers or parts respectively; and
   b. applying an effective amount of the mixture thus obtained to a tooth surface for a sufficient time to whiten a tooth.

## Claims

1. A package comprising a deformable material configured to form at least two sealed chambers, the package having
(i) a first chamber, a second chamber, and optionally additional chambers, the chambers being separated by one or more barriers which are frangible or tearable, ; and
(ii) an opening means to provide an outlet through which the mixture can be dispensed, wherein the first chamber contains a liquid solution with a viscosity below 5,000 cps comprising a protein having perhydrolase activity, the second chamber contains a carboxy donor, and the second chamber contains a peroxide source, such that upon the exertion of force on the package, one or more barriers between the chambers is compromised to an extent sufficient to permit the liquid solution to mix with the peroxide source and the carboxy donor, whereupon the liquid solution comprising a protein having perhydrolase activity catalyzes a reaction between the peroxide released by the peroxide source and the carboxy donor to form a peracid, wherein the peroxide source is a solid peroxide source selected from urea peroxide, a polyvinylpyrrolidone-hydrogen peroxide complex, sodium percarbonate, sodium perborate, and a metal peroxide, and wherein the carboxy donor is selected from a carboxylic acid and an acyl compound.

2. The package of any of the foregoing claims wherein the material in the second chamber is in the form of a powder, preferably wherein the carboxy donor comprises 1,2,3-triacetoxypropane.

3. The package of any of the foregoing claims wherein the carboxy donor is selected from one or more of (i) C₂₋₁₈ carboxylic acids, optionally substituted with hydroxy and/or C₁₋₄ alkoxy, (ii) hydrolysable and acceptable esters thereof, and (iii) mixtures thereof.

4. The package according to any of the foregoing claims wherein the second chamber contains a gellant in powder form, preferably wherein the gellant is selected from: a carbomer gallant; a polysaccharide gum; a modified food starch; an animal or fish-based gelatin; silica; and a combination of two or more thereof.

5. The package according to any of the foregoing claims wherein
a. the aqueous solution with a viscosity below 5,000 cps comprises a perhydrolase and a buffer, and
b. the second chamber contains a gellant, a peroxide, and an acetyl-containing compound, all in powder form,
such that when the frangible barrier is compromised and the contents of the chambers are allowed to mix, the peroxide and the acetyl containing compound react in the presence of the perhydrolase to form peracetic acid, in an extrudable gel formed by the liquid and the gellant.

6. The package according to any of the foregoing claims wherein the mixture of the contents of the chambers forms an orally acceptable extrudable gel, and/or wherein the mixture of the contents of the chambers is a palatable substance.

7. The package according to any of the foregoing claims wherein one or more of the chambers contains a flavoring material, and/or wherein one or more of the chambers contains a polymer which adheres to the tooth or gum surface.

8. A multi-part oral care composition comprising a first part which is physically separated from a second part during storage and combined with the second part just prior to use, wherein the parts comprise ingredients that, when combined, provide a peracid and dioxirane whitening material;
wherein the first part comprises protein having perhydrolase activity as described for any of the foregoing packages, and the second part comprises a peroxide source and a carboxy donor selected from a carboxylic acid and an acyl compound, wherein the peroxide source and the carboxy donor react in the presence of the perhydrolase to form a peracid; and
wherein the peroxide source is a solid peroxide source selected from urea peroxide, a polyvinylpyrrolidone-hydrogen peroxide complex, sodium percarbonate, sodium perborate, a metal peroxide, and a combination of two or more thereof.

9. The oral care composition according to claim 8 wherein the carboxy donor is 1,2,3-triacetoxypropane or wherein the peroxide source is urea peroxide.

10. The oral care composition according to any of claims 8-9 wherein the mixture of the ingredients from the parts forms an orally acceptable extrudable gel, and/or wherein the mixture of the ingredients from the parts is a palatable substance, and/or wherein one or more of the parts contains a flavoring material, and/or wherein one or more of the parts contains a polymer which adheres to the tooth or gum surface.

11. The oral care composition according to claim 8, wherein the first part comprises an orally acceptable ketone which forms a dioxirane upon reaction with a peracid,

12. A method of whitening teeth comprising:
a. activating a package according to claim 1 or a multi-part oral care composition according to claim 8 by combining the materials in the different chambers or parts respectively; and
b. applying an effective amount of the mixture thus obtained to a tooth surface for a sufficient time to whiten a tooth.
